# EUROPEAN PATENT APPLICATION

(11) **EP 0 956 839 A2**
(43) Date of publication of application: **17.11.1999**
(21) Application number: 99108980.6
(22) Date of filing: 06.05.1999
(51) Int. Cl.: A61F 5/44

(54) **Body-fluid drainage device**

(30) Priority: 11.05.1998 IT BO980300
(71) Applicant: Medistar S.r.l., 41037 Mirandola (IT)
(72) Inventor: Montanari, Stefano, 41100 Modena (IT)
(74) Representative: Jorio, Paolo

(57) **Abstract**

A drainage device (1) for draining body fluids withdrawn from a patient by means of a catheter (2) or similar inserted into the patient's body; the device having a collecting bag (3), and a feed tube (4) connecting the collecting bag (3) to the catheter (2); the collecting bag (3) having, internally, a drip chamber (6); the feed tube (4) being connected directly to the drip chamber (6); and the drip chamber (6) being lined with a layer (7) of bactericidal positive ions of metal salts.

## Description

The present invention relates to a body-fluid drainage device.

More specifically, the present invention relates to a device for collecting urine or any other body fluid withdrawn from a patient's body by means of a catheter or similar; to which application the following description refers purely by way of example.

As is known, currently marketed urine collecting devices comprise a body-fluid collecting bag, and a plastic feed tube having a first end connected to the collecting bag, and a second end with a connector to which the catheter is fitted, so as to feed the urine withdrawn from the patient into the collecting bag.

To prevent any bacteria inside the collecting bag from reaching the patient, collecting devices normally comprise a sterilizing member for preventing colonies of bacteria from spreading inside the collecting bag, or a one-way valve for preventing the urine from flowing back from the collecting bag to the patient.

Urine, in fact, like many other body fluids, depending on the patient's condition, is known to contain in suspension large quantities of bacteria for which the inside of the collecting bag or feed tube is an excellent environment for growth. As serious diseases may be caused by bacteria traveling back up along the feed tube into the patient's body, steps must be taken to prevent this from occurring.

Unfortunately, collecting devices of the above type have the drawback of being relatively expensive to produce and not particularly effective in preventing migration of bacteria towards the patient.

It is therefore an object of the present invention to provide a body-fluid drainage device which is cheap to produce and highly effective in preventing migration of bacteria towards the patient.

According to the present invention, there is provided a drainage device for draining body fluids withdrawn from a live organism; the drainage device comprising a holding vessel for accumulating said body fluids, and a feed tube having a first end connected to said holding vessel, and a second end connectable to said live organism; said holding vessel having, internally, a drip chamber; said feed tube being connected directly to said drip chamber; and said drainage device being characterized by said drip chamber having an inner cover layer of bactericidal material.

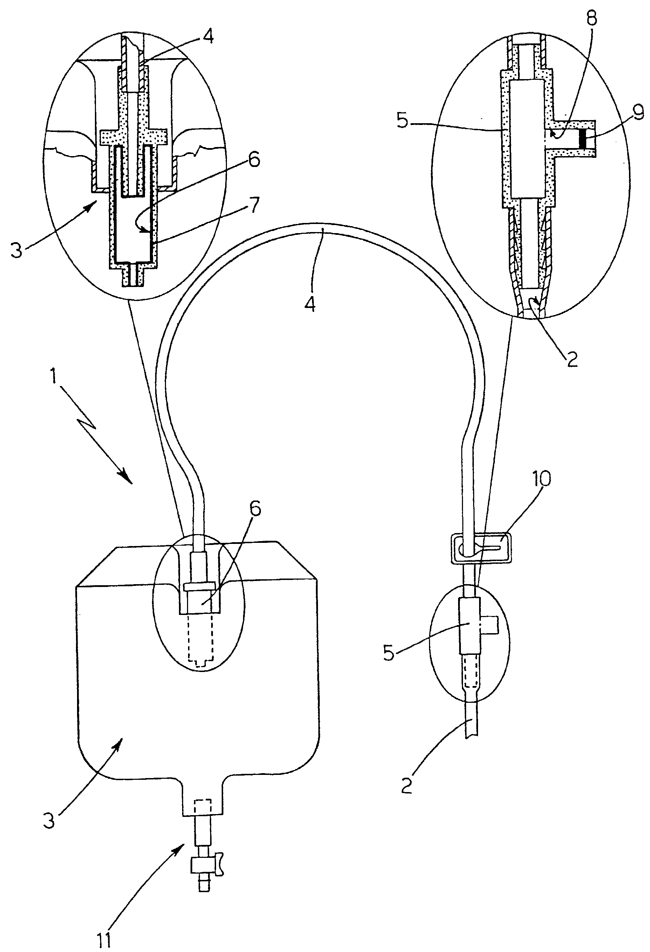

The present invention will be described with reference to the accompanying drawing, which shows a body-fluid drainage device in accordance with the teachings of the present invention.

Number 1 in the accompanying drawing indicates as a whole a body-fluid drainage device for storing body fluids, e.g. urine, withdrawn from a patient, either human or animal, using any withdrawal means, such as a catheter 2 inserted into the patient's body.

Drainage device 1 comprises a body-fluid collecting bag 3; and a feed tube 4 having a first end connected to collecting bag 3, and a second end having a fitting 5 for connection to said withdrawal means, so that the body fluids flow along feed tube 4 into collecting bag 3.

Collecting bag 3 is preferably, but not necessarily, made of plastic material, and has a drip chamber 6 inside. Feed tube 4 is also preferably, but not necessarily, made of plastic material, and is connected directly to drip chamber 6 so that the body fluids flowing inside the tube flow into collecting bag 3 via drip chamber 6.

To prevent colonies of bacteria from migrating inversely, i.e. from collecting bag 3 to the patient, drip chamber 6 has an inner cover layer 7 of bactericidal material.

The bactericidal material preferably, but not necessarily, comprises metal ions. More specifically, tests have shown the best bactericidal action to be achieved using ions of metal salts, such as zinc acetate.

In the example shown, collecting bag 3 is provided at the bottom with a drain tap 11 by which to empty collecting bag 3.

Drainage device 1 also comprises a withdrawal point by which to withdraw the body fluids flowing along catheter 2 for chemical and physical analysis.

In the example shown, the withdrawal point is formed in fitting 5, which is defined by a tubular body, the lateral wall of which comprises a through hole 8 closed in fluidtight manner by a diaphragm 9 defining the withdrawal point. Diaphragm 9 is perforated easily by a needle or similar to enable troublefree withdrawal of the body fluids from fitting 5.

In the example shown, to assist withdrawal of the body fluids from catheter 2, drainage device 1 comprises a pressure clamp 10 located along feed tube 4, downstream from fitting 5, and which is tightened onto feed tube 4 to cut off the outflow of body fluids and so fill the tube portion between pressure clamp 10 and fitting 5.

Operation of drainage device 1 is easily deducible from the foregoing description : to reach the patient, any bacteria growing inside collecting bag 3 must pass through drip chamber 6, where they are brought into contact with, and destroyed by, cover layer 7, thus safeguarding the patient from any risk of contagion.

The main advantage of drainage device 1 as described and illustrated herein is that of being of extremely straightforward design enabling rapid assembly and a considerable reduction in manufacturing cost.

A further advantage lies in drainage device 1 being free from clogging, and ensuring a high degree of inverse sterilization. The bactericidal action of cover layer 7, in fact, is greater the longer the bacteria-carrying urine from collecting bag 3 remains inside drip chamber 6.

Clearly, changes may be made to drainage device 1 as described and illustrated herein without, however, departing from the scope of the present invention.

## Claims

1. A drainage device (1) for draining body fluids withdrawn from a live organism; the drainage device (1) comprising a holding vessel (3) for accumulating said body fluids, and a feed tube (4) having a first end connected to said holding vessel (3), and a second end connectable to said live organism; said holding vessel (3) having, internally, a drip chamber (6); said feed tube (4) being connected directly to said drip chamber (6); and said drainage device (1) being characterized by said drip chamber (6) having an inner cover layer (7) of bactericidal material.

2. A device as claimed in Claim 1, characterized in that said bactericidal material comprises metal ions.

3. A device as claimed in Claim 2, characterized in that said metal ions are ions of metal salts.

4. A device as claimed in Claim 3, characterized in that said holding vessel (3) for accumulating body fluids is a body-fluid collecting bag (3).

5. A device as claimed in any one of the foregoing Claims, characterized in that said feed tube (4) comprises, at said second end, a fitting (5) connectable to withdrawal means (2) inserted into the body of said live organism.

6. A device as claimed in Claim 5, characterized in that said fitting (5) comprises a withdrawal point (9).

7. A device as claimed in Claim 6, characterized by comprising closing means (10) for closing said feed tube (4) and which are located along the feed tube (4), downstream from said fitting (5).

8. A device as claimed in Claim 4, characterized in that said metal salts comprise zinc acetate.
